# EUROPEAN PATENT APPLICATION

(11) **EP 3 020 415 A1**
(43) Date of publication of application: **18.05.2016**
(21) Application number: 14306819.5
(22) Date of filing: 17.11.2014
(51) Int. Cl.: A61K 45/06, A61K 31/407, A61P 31/04, A61K 31/185, A61K 31/198

(54) **Thiol compounds for use in bacterial infections**

(71) Applicant: Université de Fribourg, 1700 Fribourg (CH)
(72) Inventor: Nordmann, Patrice, 1700 Fribourg (CH); Poirel, Laurent, 1700 Fribourg (CH)
(74) Representative: Cabinet Plasseraud

(57) **Abstract**

A compound selected in the group consisting in (RS)-2,3-bis(sulfanyl)propane-1-sulfonic acid or one of his salts for use in a method of treating a bacterial infection, pharmaceutical compositions comprising (RS)-2,3-bis(sulfanyl)propane-1-sulfonic acid or one of his salts, and a method for inhibiting ß-lactamases of bacteria in warm-blooded animals, comprising administering to warm-blooded animals a ß-lactamase inhibiting effective amount of at least (RS)-2,3-bis(sulfanyl)propane-1-sulfonic acid or one of his salts.

## Description

The present invention relates to thiol compounds for use in a method of treating a bacterial infection and compositions comprising a thiol compound and a ß-lactam antibiotic.

Current antibacterial chemotherapy for treating multi-drug resistant bacteria is becoming increasingly inadequate due to the rise of clinical resistance, mainly related to the spread of genes encoding ß-lactam resistance. ß-lactams comprise penicillins, cephalosporins, carbapenems and monobactams, all sharing in common a strained ß-lactam ring that acts as a suicide substrate, acylating the key transpeptidase enzymes involved in cell wall formation. Carbapenems which, until now, were the most effective antibiotics for treating Gram-negative infections, are becoming increasingly ineffective.. Clinically-available carbapenems worldwide are mostly imipenem, ertapenem and meropenem. Among the ß-lactam antibiotics, they possess the broadest spectrum of activity. Those carbapenemases are proteins that degrade not only carbapenems but also many other ß-lactams molecules. Their exact spectrum of activity depends of their structure.

Three main classes of clinically-significant carbapenemases have been reported, i.e. Ambler class A, D and B enzymes (Nordmann et al., 2011). Ambler class A and D enzymes are serine proteins whereas class B enzymes are metallo-enzymes.

The above enzymes are named metallo-ß-lactamases (MBLs) because they use zinc ion catalysis and water instead of an active serine to hydrolyse the ß-lactam ring of ß-lactam molecules. A high diversity of MBLs is known, including in particularly NDM, VIM, IMP, SPM, GIM, AIM, and SIM (Cornaglia et al. 2011). Those MBLs share a small number of conserved motifs, but otherwise they show significant sequence diversity and have thus been classified into three subclasses: subclass B1 included BcII from *Bacillus cereus,* CfiA from *Bacteroides fragilis,* IMP-1 and VIM-1 from *Pseudomonas aeruginosa,* NDM-1 and relatives (ten variants identified so far) from *Enterobacteriaceae, A. baumannii, P. aeruginosa,* and *blaB* from *Chryseobacterium meningosepticum;* subclass B2 essentially consists of enzymes from *Aeromonas* strains and subclass B3 includes enzymes such as the L1 enzyme from *Stenotrophomonas maltophilia* along with GOB-1 from *Chryseobacterium meningosepticum.* NDM and VIM enzymes have become important in threatening pathogens whereas the other MBLs are spreading relatively locally (Nordmann *et al.* 2011). Several MBLs have been also identified as natural resistance factors to carbapenems such as in *Stenotrophomonas maltophilia* (another Gram-negative species) and in *Flavobacterium* sp.

IMP-type enzymes were first reported in *Enterobacteriaceae* and *Pseudomonas aeruginosa* in Asia (Japan) (Cornaglia *et al.* 2011). Then IMP and VIM-types enzymes have been identified worldwide including in Europe. VIM-2 has identified in particularly in *P. aeruginosa* where it remains currently the most widely distributed carbapenemase. Since 2008, NDM enzymes are emerging the frontline of the antibiotic resistance scene. NDM-1 (Nordmann *et al.,* 2011). Subsequently the spread of NDM producers worldwide has been identified rapidly. Several regions may be considered now as endemic for NDM producers such as Indian subcontinent, the Arabic peninsula, the Middle East and the Balkan states (Nordmann *et al* 2011). The *bla*NDM-like genes have been identified not only in *Enterobacteriaceae* but also in *A. baumannii* and *P. aeruginosa* in clinical and environmental isolates.

The hydrolytic activity of metallo-enzymes includes all ß-lactams except aztreonam (Cornaglia *et al.* 2011). However, many of the MBL producers are also resistant to aztreonam since most of those producers possess additional mechanisms of resistance to ß-lactams.

Ten alleles of the *bla*NDM gene are known that sharing 99.4% amino acid identity. Some the NDM proteins (NDM-4, NDM-7) have extended hydrolytic activity towards carbapenems as compared to the reference NDM-1 protein (Nordmann & Poirel 2014). Similarly, a total of 39 different VIM variants are known.

The drugs of last resort for treating infections due to carbapenemase producers include polymyxins (such as colistin), tigecyline, fosfomycin, and rarely several aminoglycosides. These options are generally limited by a lack of clinical data on efficacy, as well as by concerns about pharmacokinetic and toxicity of several of those molecules.

Colistin is one of the first line agents for treating those infections. Although introduced in the 1950's, its use was abandoned for aminoglycosides. Many data on safety and efficacy of colistin are based therefore on older studies. Neurotoxicity and nephrotoxicity are the two main concerns with colistin. Dosing issue have not been completely clarified and outbreaks of colistin-resistant carbapenemase producers in Enterobacteriaceae have been already reported.

Tigecycline is a new antibiotic of the tetracycline class. Like colistin, there is no oral form of this antibiotic for systemic infections. The main side effect of tigecycline is nausea but pancreatitis has been reported. The efficacy of tigecycline is limited by increasing reports of acquired resistance in vivo and weak tissue diffusion (kidney and urines). It is not therefore recommended for treating pneumonia and urinary tract infections.

Fosfomycin has limited activity against Gram negative systemic infections. In addition, only its oral formula is available in many countries including the USA.

Adding a ß-lactamase inhibitor to a ß-lactam antibiotic is a highly validated and commercially successful approach to "rescue" an antibiotic failing in the face of emerging resistance and also to extend the activity spectrum to bacterial species not originally susceptible to the original compound (Geddes *et al,* 2007; Kim *et al.,* 2010; Novelli *et al.* 1989). Such combinations were found to be active against many class A ß-lactamases (amoxicillin/clavulanate ticarcillin/clavulanate, ampicillin/sulbactam, piperacillin/tazobactam). Attempts to discover a MBL inhibitor drug that may be used safely in human have failed. Most known MBL inhibitors contain a zinc-binding moiety such as narylsulfonylhydrazones, thiomandelic acids, or thiosemicarbazides (Faridoon *et al.,* 2012; Ishii *et al* 2013; *Kim et al.,* 2012; Livermore *et al.,* 2013, Olsen *et al.,* 2006. MBL inhibition was achieved, but poor selectivity and poor drug-like properties prevented in vitro antimicrobial synergy with a carbapenem. Inhibitors of several MBLs have been identified in the group of dicarboxylic acids such as succinic, maleic and phtalic acids. Based on a dicarboxylic acid template, they were shown to bind to the zinc atom in the active site of IMP-1. One of those compounds which is ME1071, a maleic acid derivative, has completed Phase I clinical trials. ME1071 shows 10- to 100-fold greater affinity than imipenem or biapenem for IMP-1 and VIM-2. However, it was recently reported that the affinity of ME1071 for NDM-1 is considerably weaker (Ki = 24mM) (Livermore *et al.* 2013).

No efficient treatment is available for treating Gram-negative bacteria producing carbapenemases. Therefore the purpose of the present invention is to contribute to development of antibiotics for treating infections due to multidrug resistant bacteria producing ß-lactamases, particularly carbapenemases of the metallo-enzyme class.

DMPS, (RS)-2,3-bis(sulfanyl)propane-1-sulfonic acid, formerly known as (R,S)-2,3-dimercaptopropane- 1-sulfonic acid, is the compound with the formula HSCH₂ -CH(SH)-CH₂SO₃H. DMPS is used in the pharmaceutical field as a treatment for heavy metal toxicity and is marketed in capsules under the trade name of Dimaval® as hard capsules and as a solution for injection (Aposhian, 1983). The parenteral form can be administered via the intravenous and intramuscular routes. The toxicology, metabolism, pharmacokinetic profile, safety and therapeutic efficacy of DMPS are well known from extensive clinical trials.

The inventors found that (RS)-2,3-bis(sulfanyl)propane-1-sulfonic acid and his salts such as the sodium salt possess very useful original pharmacological properties; they are endowed in particular with remarkable ß-lactamase inhibitor properties, more particularly remarkable carbapenemase inhibitor properties. Accordingly, DMPS is able to inhibit the inactivation of beta-lactam antibiotics such that said antibiotics recover anti bacterial properties.

These properties are illustrated below in the experimental section. They justify the use of DMPS or one of his salts, preferably his sodium salt as ß-lactamase, more particularly as carbapenemase inhibitor drugs in antibiotherapy.

Accordingly, the present invention relates to (RS)-2,3-bis(sulfanyl)propane-1-sulfonic acid and his salts for use in a method of treating bacterial infections, and more particularly in a method of treating infections due to multidrug resistant bacteria producing ß-lactamases, more particularly producing carbapenemases of the metallo-enzyme class.

The present invention also relates to the use of (RS)-2,3-bis(sulfanyl)propane-1-sulfonic acid and his salts, for manufacturing a medicament for use as ß-lactamase inhibitor or in a method of treating bacterial infections, and more particularly in a method of treating infections due to multidrug resistant bacteria producing ß-lactamases, more particularly producing carbapenemases of the MBL class. Bacterial species that will be targeted as MBL producers are in particularly *Enterobacteriaceae, Pseudomonas aeruginosa* and *Acinetobacter baumannii.*

(RS)-2,3-bis(sulfanyl)propane-1-sulfonic acid and his salts are preferably used in a method of treating infections due to multidrug resistant bacteria producing carbapenemases of the metallo-enzyme class.

Of course, (RS)-2,3-bis(sulfanyl)propane-1-sulfonic acid and his salts should be used in association with a suitable ß-lactam antibiotic compound, sequentially or preferably concomitantly.

The present invention further relates to a method for inhibiting ß-lactamases of bacteria in warm-blooded animals, comprising administering to warm-blooded animals a ß-lactamase inhibiting effective amount of at least (RS)-2,3-bis(sulfanyl)propane-1-sulfonic acid or one of his salts, preferably his sodium salt.

The present invention further relates to a method for treating an infection by ß-lactamase-producing bacteria in warm-blooded animals, particularly an infection due to multidrug resistant bacteria producing ß-lactamases, more particularly producing carbapenemases of the metallo-enzyme class in warm-blooded animals, comprising administering to warm-blooded animals a ß-lactamase inhibiting effective amount of at least (RS)-2,3-bis(sulfanyl)propane-1-sulfonic acid or one of his salts, preferably his sodium salt, and a ß-lactam antibiotic compound sensitive to ß-lactamase.

The compounds can be administered orally, rectally, preferably parenterally and the usual daily dose is 0.1 to 900 mg/kg, preferentially 30 mg/kg depending on the specific compound, the infection and the route of administration. For example, intravenous administration of 30 mg/kg daily of sodium salt of (RS)-2,3-bis(sulfanyl)propane-1-sulfonic acid may be useful for treatment of a septicemia due to *Klebsiella pneumoniae* using imipenem (2 g daily) as ß-lactam companion molecule.

The antibiotic compound and (RS)-2,3-bis(sulfanyl)propane-1-sulfonic acid or one of his salts may be administered separately or in a single pharmaceutical composition.

These properties also justify the use of (RS)-2,3-bis(sulfanyl)propane-1-sulfonic acid or one of his salts in a pharmaceutical composition further comprising a ß-lactamase sensitive antibiotic compound.

The novel antibiotic pharmaceutical compositions of the invention are comprised of an effective amount of at least (RS)-2,3-bis(sulfanyl)propane-1-sulfonic acid or one of his salts, preferably his sodium salt, a ß-lactam antibiotic compound sensitive to ß-lactamase and optionally but preferably an inert pharmaceutical carrier or excipient. The compositions may be, for example in the form of tablets, capsules, granules, or injectable solutions or suspensions.

Examples of suitable excipients are talc, gum arabic, lactose, starch, magnesium stearate, cocoa butter, aqueous and non-aqueous vehicles, fatty substances of animal or vegetable origin, paraffin derivatives, glycols, various wetting, dispersing or emulsifying agents and preservatives.

(RS)-2,3-bis(sulfanyl)propane-1-sulfonic acid or one of his salts may particularly be formulated for parenteral administration (e.g., by injection, for example bolus injection or continuous infusion). The active ingredients may be presented in unit dose form in ampoules, pre-filled syringes small volume infusion or in multi-dose containers with or without an added preservative. Suitable pharmaceutical forms comprise suspensions, solutions, or emulsions in oily or aqueous vehicles. Examples of oily or non-aqueous carriers, diluents solvents or vehicles include propylene glycol, polyethylene glycol, vegetable oils (e.g., olive oil, and injectable organic esters (e.g., ethyl oleate), and may contain adjuvant agents such as preserving, emulsifying or suspending, wetting, stabilizing and/or dispersing agents. The active ingredients may also be in powder form, obtained by aseptic isolation of sterile solid or by lyophilization from solution for constitution before use with a suitable vehicle, such as sterile, pyrogen-free water.

The novel antibiotic pharmaceutical compositions of the invention are useful for example in the curative treatment of infections by bacteria such as Enterobacteriaceae (such as *Escherichia coli, Klebsiella pneumoniae, Klebsiella oxytoca, Morganella morganii, Serrratia marcescens*, *Proteus mirabilis, Enterobacter cloacae, Enterobacter aerogenes*), and Gram-negative non fermenters (such as *Pseudomonas aeruginosa, Stenotrophomonas maltophilia Acinetobacter baumannii*)

The usual dose, which varies depending on the subject and the infection in question, depends on the nature and the amount of the antibiotic compound (imipenem - meropenem- ertapenem- biapenem - doripenem).

Among the preferred pharmaceutical compositions of the invention are those wherein (RS)-2,3-bis(sulfanyl)propane-1-sulfonic acid or one of his salts, preferably his sodium salt, is associated with one or more ß-lactam antibiotic compounds sensitive to ß-lactamase, preferably one or more carbapenems, more preferably one or more of the following antibiotic compounds:
- imipenem
- meropenem
- ertapenem
- biapenem
- doripenem
- panipenem
- razupenem
- tebipenem
- lenapenem
- tomopenem, preferably
- imipenem
- meropenem
- ertapenem
- biapenem or
- doripenem
- panipenem, more preferably
- imipenem or
- ertapenem.

Any other ß-lactam molecules may be associated with (RS)-2,3-bis(sulfanyl)propane-1-sulfonic acid or one of his salts such as carboxypenicillins (ticarcillin,..) ureidopenicillins (piperacillin...) or cephalosporins (cefotaxime, ceftazidime, cefepime, cefpirome, ceftriaxone) or moxalactam or aztreonam with or without a carbapenem.

In addition, for an enhanced effect, (RS)-2,3-bis(sulfanyl)propane-1-sulfonic acid or one of his salts and the above combinations of ß-lactam molecules with (RS)-2,3-bis(sulfanyl)propane-1-sulfonic acid or one of his salts may further be associated with a polyacid chelating compound, preferably a polyacetic compound, particularly ethylenediaminetetraacetic acid, abbreviated as EDTA. The chelating compounds are preferably used as conjugates such as calcium-ethylenediaminetetraacetate.

The present invention also relates to a process for preparing a pharmaceutical composition described above, characterized in that, according to methods known per se, the antibiotic ingredient and (RS)-2,3-bis(sulfanyl)propane-1-sulfonic acid or one of his salts, preferably his sodium salt are mixed with optional acceptable excipients, particularly pharmaceutically acceptable excipients.

The scope of the invention can be understood better by referring to the examples given below, the aim of which is to explain the advantages of the invention.

### EXAMPLES

### EXAMPLE 1: Tablets

Tablets were prepared containing 400 mg of (RS)-2,3-bis(sulfanyl)propane-1-sulfonic acid sodium salt, 500 mg of imipenem and sufficient excipient of lactose, starch, talc and magnesium stearate to obtain a final weight of 2 g.

### EXAMPLE 2: Tablets

Tablets were prepared containing 400 mg of (RS)-2,3-bis(sulfanyl)propane-1-sulfonic acid sodium salt, 500 mg of ertapenem and sufficient excipient of lactose, starch, talc and magnesium stearate to obtain a final weight of 2 g.

### EXAMPLE 3: Powder for parenteral injections

Vials were prepared containing 400 mg of (RS)-2,3-bis(sulfanyl)propane-1-sulfonic acid sodium salt and 500 mg of imipenem. An aqueous solvent was provided in a separate vial.

### EXPERIMENTAL DATA

(RS)-2,3-bis(sulfanyl)propane-1-sulfonic acid sodium salt was evaluated as inhibitor of MBLs using a panel of strains producing the most clinically significant carbapenemases of the metallo-enzyme group.

DMPS was evaluated as inhibitor of MBLs using a panel of strains producing the most clinically significant carbapenemases of the metallo-enzyme group. Minimum inhibitory concentrations (MICs) of carbapenems were determined in-vitro with strains (reference and clinical) producing main types of class B carbapenemases which are IMP, VIM, NDM, GIM and SPM with or without DMPS at various concentrations. The efficacy of DMPS at various concentrations with the two main carbapanemem molecules, meropenem and imipenem, used for treating Gram negatives infections was analyzed. The studied strains were clinical isolates (to mirror multidrug resistance in clinical isolates) for which a precise ß-lactamase gene content is known (Table 1). The strains were NDM, VIM-, IMP, SPM and GIM and producers as well as control of a non-MBL producer (OXA-48). The selected concentration of DMPS was 1-fold, 3-fold (0.3 mM) and 30-fold (3 mM) that of the human peak serum concentration after a single IV dose of DMPS (Aposhian,1983).

The MICs of carbapenems were performed with Zn (5mg/L) to ensure full in-vivo expression of metallo-enzymes. Inhibitory properties of DMPS were also analyzed in-vitro by UV spectrophotometry using purified MBLs and carbapenems as susbtrates.

*E. coli* TOP10 pCR2.1-(*P*_{NDM-1}-*bla*_{NDM-1}-*ble*_{MBL}), expressing the *bla*_{NDM-1} gene with its original promoter (*P*_{NDM-1}) cloned into a pTOPO multicopy plasmid and expressed in *E. coli* TOP10 reference strain (Nordmann *et al.* 2012) was used for preliminary in-vitro antibacterial activity assays in order to determine the most efficient concentrations of DMPS against NDM producers. MICs of imipenem was determined for *E. coli* TOP10 pCR2.1-(*P*_{NDM-1}-*bla*_{NDM-1}-*ble*_{MBL}) in the presence of 0 to 30 mM of DMPS by using the broth microdilution method in zinc-complemented M9CA minimum medium (SD7025, Euromedex, France). M9CA minimal medium was used preferably to the standard Mueller Hinton broth in order to determine precisely the concentration of zinc in broth (ZnSO₄, Sigma Aldrich, France, 5 mg/L (final concentration).

A 100 mM DMPS stock solution (Sigma Aldrich, France) (pH 5.0) was prepared in 0.2 M NaOH as recommended (Maiorino et al. 1990) and pH was neutralized to pH 7.0 with NaHCO₃ Na₂Ca-EDTA (Sigma Aldrich, France) dissolved in water.

### Results

Determination of MIC values of imipenem for *E. coli* TOP10 pCR2.1-(*P*_{NDM-1}-*bla*_{NDM-1}*-bl*e_{MBL}) showed that the maximal inhibition efficiency was 50 mM (data not shown). The reported serum concentration after IV administration reaches ca. 110 µM. DMPS is mostly eliminated by urines and its derivatives as well may have some chelator properties (Aposhian, 1983).

They values varied among isolates producing a same carbapenemase type. DMPS at 3 mM lowered most significantly the MIC values of meropenem for the metallo-enzymes producers. In addition real in-vivo efficacy of DMPS may be higher than that observed in vitro since this molecule is susceptible, in-vivo, to oxydation.

| Enterobacteriacae | | | | | |
|---|---|---|---|---|---|
| Species | Enzyme | IPM µg/ml | IPM+ DMPS 0.1mM | IPM+ DMPS 0.3 mM | IPM+ DMPS 3 mM |
| E. coli | NDM-1 | 8 | 8 | 8 | 2 |
| E. coli | NDM-1 | 8 | 8 | 4 | 2 |
| E. coli | NDM-1 | 8 | 8 | 8 | <0.5 |
| K. pneumoniae | NDM-1 | 64 | 32 | 32 | 2 |
| K. pneumoniae | NDM-1 | 64 | 32 | 32 | 8 |
| E. coli | NDM-4 | 64 | 64 | 64 | 8 |
| E. coli | NDM-5 | 64 | 32 | 32 | 8 |
| E. cloacae | GIM-1 | 8 | 4 | 2 | 2 |
| C. freundii | VIM-2 | 2 | 2 | 2 | 2 |
| K. pneumoniae | VIM-4 | 64 | 64 | 64 | 8 |
| K. pneumoniae | IMP-4 | 16 | 16 | 16 | 0.5 |
| K. pneumoniae | IMP-8 | 8 | 8 | 8 | 2 |
| K. pneumoniae | OXA-48 | 32 | 32 | 32 | 32 |
| | | | | | |

| Species | Enzyme | MEM µg/ml | MEM + DMPS 0.1mM | MEM + DMPS 0.3mM | MEM + DMPS 3mM |
|---|---|---|---|---|---|
| E. coli | NDM-1 | 32 | 32 | 32 | <0.5 |
| E. coli | NDM-1 | 16 | 16 | 8 | <0.5 |
| E. coli | NDM-1 | 32 | 32 | 16 | <0.5 |
| K. pneumoniae | NDM-1 | 128 | 128 | 128 | 4 |
| K. pneumoniae | NDM-1 | 64 | 64 | 64 | 32 |
| E. coli | NDM-4 | 64 | 64 | 64 | 8 |
| E. coli | NDM-5 | 64 | 64 | 64 | 4 |
| E. cloacae | GIM-1 | 16 | 8 | 4 | 0.25 |
| C. freundii | VIM-2 | 0.06 | 0.06 | 0.06 | <0.06 |
| K. pneumoniae | VIM-4 | 32 | 32 | 32 | 2 |
| K. pneumoniae | IMP-4 | 16 | 16 | 8 | 0.25 |
| K. pneumoniae | IMP-8 | 2 | 2 | 2 | 0.06 |
| K. pneumoniae | OXA-48 | 32 | 32 | 32 | 32 |
| | | | | | |
| Non-Enterobacteriacae | | | | | |

| Species | Enzyme | IPM µg/ml | IPM+ DMPS 0.1mM | IPM+ DMPS 0.3mM | IPM+ DMPS 3mM |
|---|---|---|---|---|---|
| A. baumannii | NDM-1 | 128 | 128 | 128 | 128 |
| A. baumannii | NDM-1 | 128 | 128 | 128 | 128 |
| P. aeruginosa | NDM1 | 1,024 | 1,024 | 512 | 512 |
| P. aeruginosa | GIM-1 | >1,024 | >1,024 | >1,024 | >1,024 |
| P. aeruginosa | VIM-1 | 128 | 128 | 128 | 64 |
| P. aeruginosa | VIM-2 | 1,024 | 1,024 | 1,024 | 512 |
| P. putida | VIM-2 | 128 | 128 | 128 | 64 |
| P. aeruginosa | IMP-13 | 128 | 64 | 32 | 64 |
| A. baumannii | IMP-4 | 64 | 64 | 64 | 8 |
| P. aeruginosa | SPM1 | 1,024 | 1,024 | 512 | 256 |
| K. pneumoniae | OXA-48 | 32 | 32 | 32 | 32 |
| | | | | | |

| Species | Enzyme | MEM µg/ml | MEM + DMPS 0.1mM | MEM + DMPS 0.3mM | MEM + DMPS 3mM |
|---|---|---|---|---|---|
| A. baumannii | NDM-1 | 256 | 256 | 256 | 128 |
| A. baumannii | NDM-1 | 256 | 256 | 256 | 128 |
| P. aeruginosa | NDM1 | 256 | 256 | 256 | 256 |
| P. aeruginosa | GIM-1 | 1,024 | 1,024 | 1,024 | 512 |
| P. aeruginosa | VIM-1 | 256 | 256 | 256 | 256 |
| P. aeruginosa | VIM-2 | 1,024 | 1,024 | 1,024 | 1,024 |
| P. putida | VIM-2 | 128 | 128 | 128 | 64 |
| P. aeruginosa | IMP-13 | 64 | 64 | 64 | 16 |

Table 1. Minimum Inhibitory values of imipenem (IPM) with or without various concentration of (RS)-2,3-bis(sulfanyl)propane-1-sulfonic acid sodium salt (DMPS° for several metallo-carbapenemase producers as well as and OXA-48 producer used as a control.

DMPS was more active against *Enterobacteriacae* than against *P. aeruginosa* and *A. baumannii.* Those latter results may be due to difference in the outer-membrane permeability structure which is well-known to be less permeable for *P. aeruginosa* and *A. baumannii* than for *Enterobacteriaceae*

In-vitro inhibition of several metallo-ß-lactamases by DMPS was assessed using a purified fraction of MBL enzymes, obtained as previously described for NDM-4 (Nordmann *et al.* 2012) with imipenem 100 µM as substrate. The concentration of DMPS suppressing 50% of metallo-ß-lactamases (IC₅₀) was found to be low for the metallo-ß-lactamases tested including NDM-4 that possesses an extended spectrum of carbapenemase activity (Table 2). Those results show the high inhibitory property of DMPS against metallo-ß-lactamases.

**Table 2. Inhibitory concentration 50 (IC 50 [µM]) of (RS)-2,3-bis(sulfanyl)propane-1-sulfonic acid sodium salt for several metallo-carbapenemases and for the non-metallo-enzyme KPC-3 used as a negative control.**

| | |
|---|---|
| IMP-1 | 70 |
| NDM-1 | 80 |
| NDM-4 | 110 |
| SPM-1 | 110 |
| VIM-1 | 180 |
| KPC-3 | >10,000 |

In conclusion, (RS)-2,3-bis(sulfanyl)propane-1-sulfonic acid or one of his salts allows treating mammalian, particularly human infections due to bacteria producing at least of several types of metallo-ß-lactamases.

### Conclusion

(RS)-2,3-bis(sulfanyl)propane-1-sulfonic acid and his salts of the present invention, as shown in Tables 1/2, have the effect of recovering the antibacterial activities of imipenem and meropenem against MBL-producing bacteria of which therapy has hitherto been regarded difficult in the medical field. Thus, (RS)-2,3-bis(sulfanyl)propane-1-sulfonic acid and his salts are useful as MBL inhibitors for the combination with ß-lactam drugs and the like.

### References

1. Aposhian, H. V. 1983. DMSA and DMPS water soluble antidotes for metal poisoning. Ann Rev Pharmacol Toxicol. 23; 193-215
2. Cornaglia, G., H. Giamarellou, and G. M. Rossolini. 2011. Metallo-ß-lactamases: a last frontier for ß-lactams. Lancet Infect Dis. 11:381-93.
3. Faridoon H., M. Waleed , P. Vella, N. Ul Islam, D.L. Ollis, G. Schenk, and R. McGeary 2012. 3-Mercapto-1,2,4-triazoles and N-acylated thiosemicarbazides as metallo-β-lactamase inhibitors. Bioorganic Med Chem Lett 22:380-386
4. Geddes, A.M., K.P. Klugman, and G.N. Rolinson. 2007. Introduction: historical perspective and development of amoxicillin/clavulanate. Internation J Antimicrob Agents 30(Suppl. 2) : S109-S112.
5. Ishii, Y., M. Eto, M. Yoko, T. Kazuhir, and Y. Keizo. 2010. In vitro potentiation of carbapenems with ME1071, a novel metallo-β-lactamase inhibitor, against metallo-β-lactamase-producing Pseudomonas aeruginosa clinical isolates. Antimicrob Agents Chemotherapy 54:3625-3629.
6.Kim, S.-K., C.L. Sims, S. E. Wozniak, S. H. Drude, D. Whitson, and R. W. Shaw. 2010. Antibiotic resistance in bacteria : novel metallo-enzyme inhibitors. Chem Biol Drug Des 74 :343-348.
7. Livermore, D. M., S. Mushtaq, A. Morinaka, T. Ida, K. Maebashi, and R. Hope. 2013. Activity of carbapenems with ME1071 (disodium 2,3-diethylmaleate) against Enterobacteriaceae and Acinetobacter spp. with carbapenemases, including NDM enzymes. J Antimicrob Chemother. 68:153-158.
8. Nordmann, P., A.E. Boulanger, and L. Poirel . 2012. NDM-4 metallo-β-lactamase with increased carbapenemase activity from Escherichia coli. Antimicrob Agents Chemother. 56:2184-2186.
9. Nordmann P, Girlich D, and L. Poirel. 2012. Detection of carbapenemase producers in Enterobacteriaceae by use of a novel screening medium. J Clin Microbiol 50:2761-2766.
10. Nordmann, P., T. Naas, and L. Poirel. 2011. Global spread of carbapenemase-producing Enterobacteriaceae. Emerg. Infect. Dis. 17:1791-1798.
11. Nordmann, P., and L. Poirel. 2014. The difficult-to-control spread of carbapenemase-producing Enterobacteriacae. Clin Microb Infect, in press
12. Nordmann, P., L. Poirel, T. R. Walsh, and D. M. Livermore. 2011. The emerging NDM carbapenemases. Trends Microbiol. 19:588-595.
13. Drawz SM., Bonomo RA. 2010. Three Decades of B-Lactamase Inhibitors. Clin Microbiol Rev. 23: 160-201.
14. Olsen, L., S. Jost, H.W. Adolph, L. Pettersson, L. Hemmingsen, and F.S. Jorgensen. 2006. New leads of metallo-β-lactamase inhibitors from structure-based pharmacophore design. Bioorganic Med Chemistry 14:2627-2635.

## Claims

1. (RS)-2,3-bis(sulfanyl)propane-1-sulfonic acid or one of his salts, for use in a method of treating a bacterial infection.

2. A compound according to claim 1, **characterized in that** the compound is the sodium salt of (RS)-2,3-bis(sulfanyl)propane-1-sulfonic acid.

3. A compound according to claim 1 or 2, **characterized in that** the bacterial infection is due to one or more multidrug resistant bacteria producing carbapenemases of the metallo-enzyme class.

4. A pharmaceutical composition comprising a compound selected in the group consisting in RS)-2,3-bis(sulfanyl)propane-1-sulfonic acid and his salts, a ß-lactam antibiotic compound sensitive to ß-lactamase and optionally an inert pharmaceutical carrier or excipient.

5. A pharmaceutical composition according to claim 4, **characterized in that** the compound is the sodium salt of (RS)-2,3-bis(sulfanyl)propane-1-sulfonic acid.

6. A pharmaceutical composition according to claim 4 or 5, **characterized in that** the ß-lactam antibiotic compound sensitive to ß-lactamase is a carbapenem compound.

7. A pharmaceutical composition according to any one of claims 4 to 6, **characterized in that** the ß-lactam antibiotic compound sensitive to ß-lactamase is selected from the group consisting in imipenem and ertapenem.

8. A pharmaceutical composition according to any one of claims 4 to 7, **characterized in that** it further comprises a polyacid chelating compound.

9. A pharmaceutical composition comprising
- (RS)-2,3-bis(sulfanyl)propane-1-sulfonic acid or one of his salts as defined in one of claims 1 to 3
- a polyacid chelating compound.

10. A method for inhibiting ß-lactamases of bacteria in warm-blooded animals, comprising administering to warm-blooded animals a ß-lactamase inhibiting effective amount of at least (RS)-2,3-bis(sulfanyl)propane-1-sulfonic acid or one of his salts.

11. A method of treatment of an infection by ß-lactamase-producing bacteria in warm-blooded animals, comprising administering to warm-blooded animals a composition comprising a ß-lactamase inhibiting effective amount of at least (RS)-2,3-bis(sulfanyl)propane-1-sulfonic acid or one of his salts and a ß-lactam antibiotic compound sensitive to ß-lactamase.
